# DEMANDE DE BREVET EUROPEEN

(11) **EP 0 965 333 A2**
(43) Date de publication de la demande: **22.12.1999**
(21) Numéro de dépôt: 99400409.1
(22) Date de dépôt: 19.02.1999
(51) Int. Cl.: A61K 7/48

(54) **Nouvelle formulation topique à base de rétinaldehyde**

(30) Priorité: 19.02.1998 FR 9802012
(71) Demandeur: Dewandre, Luc, 74400 Chamonix (FR)
(72) Inventeur: Dewandre, Luc, 74400 Chamonix (FR)
(74) Mandataire: Burtin, Jean-François

(57) **Abrégé**

L'invention se rapporte au domaine de la dermato-cosmétologie.

Elle a plus précisément pour objet la réalisation d'une formulation cosmétologique particulière destinée à l'application topique d'un dérivé de la Vitamine A qui consiste en ce que la formulation comporte une phase continue à consistance de gel et une phase discontinue formée de microcapsules aisément frangibles renfermant une quantité déterminée de rétinaldéhyde, dispersée d'une manière homogène dans le gel de la phase continue.

Utilisation pour les soins de la peau et pour prévenir ou corriger les signes de vieillissement cutané.

## Description

La présente invention se rapporte au domaine de la chimie et plus particulièrement à celui de la dermato-cosmétologie.

Elle a plus précisément pour objet la réalisation d'une formulation galénique particulière destinée à l'application topique d'un dérivé de la Vitamine A.

Elle concerne spécifiquement une formulation cosmétologique destinée à l'usage topique, à base de rétinaldéhyde caractérisée en ce que la formulation comporte une phase continue à consistance de gel et une phase discontinue formée de microcapsules aisément frangibles renfermant une quantité déterminée de rétinaldéhyde, dispersée d'une manière homogène dans le gel de la phase continue.

On connaît l'intérêt du rétinaldéhyde ou rétinal comme molécule pivot des retinoïdes naturels amenant la production physiologique d'acide rétinoïque ou de rétinol ou de ses esters selon l'environnement enzymatique local. L'utilisation locale de crème à base de rétinaldéhyde a prouvé son plein intérêt pour le soin des peaux altérées ainsi que pour prévenir ou corriger les signes de vieillissement cutané.

Ainsi ces crèmes permettent l'atténuation des rides et des ridules sur le visage, la diminution de la rugosité de la peau, l'uniformisation et l'augmentation de l'aspect lumineux du teint. Elles conviennent également pour combattre la séborrhée.

On sait également que la tolérance cutanée aux préparations à base de rétinaldéhyde est sensiblement meilleure que celle des préparations à base d'acide rétinoïque du fait que la transformation en acide rétinoïque n'est que partielle.

Les rétinoïdes utilisés en dermatologie sont classiquement l'acide rétinoïque tout trans et ses analogues tels que l'acide 13 cis rétinoïque, l'acitrétine, l'acide arotinoïque etc... qui sont des stéréoïsomères et possèdent une réelle activité biologique. Ils sont cependant pourvus d'effets secondaires importants qu'ils soient utilisés par voie systémique ou par voie topique et provoquent notamment une forte irritation locale.

On a trouvé maintenant que les formulations selon l'invention pouvaient être préparées d'une manière avantageuse sous forme de gel, celui-ci présentant des possibilités d'application beaucoup plus agréables qu'une crème et ne laissant pas l'impression d'une fraction résiduelle sur la peau. Les gels ont une fluidité importante et s'étalent à l'aide de la main d'une manière beaucoup plus extensive que les crèmes de l'art antérieur. En outre la formulation sous forme de gel n'avait pas été envisagée jusqu'alors car le rétinaldéhyde est une molécule susceptible de se dégrader à la lumière ou en présence d'eau. C'est la raison pour laquelle il est apparu approprié d'insérer dans une ou plusieurs microcapsules spéciales des quantités déterminées de rétinaldéhyde sans que celui-ci soit susceptible de s'altérer.

D'une manière préférée les compositions dermatologiques et/ou cosmétologiques selon la présente invention contiennent de 0,01 à 5 % en poids de rétinaldéhyde par rapport à la composition totale.

Les microcapsules utilisées dans le cadre de la présente invention sont de dimensions très réduites s'échelonnant de 400 à 800 microns. Ces microcapsules ont également la caractéristique de s'écraser par frottement de telle sorte que l'application du gel de base contenant ces microcapsules a pour effet de provoquer l'éclatement des microcapsules dans le gel et d'assurer ainsi au moment de l'emploi la dispersion du rétinaldéhyde dans le gel. Ainsi le rétinaldéhyde protégé jusque là dans des microcapsules peut être appliqué sans risque d'altération.

Le gel de base est constitué d'un agent gélifiant commercialisé sous la dénomination SEPIGEL 305 à la dose de 2 à 5 % dans l'eau. Ce gel peut être additionné de parfums, d'agents de conservation comme des parabens ou d'agent antioxydant comme le BHT ou le BHA.

Les microcapsules utilisées dans le cadre de la présente invention sont celles commercialisées sous le nom de Thalasphères SW7 par la Société Coletica. Elles contiennent de 100 à 500 microgrammes de rétinaldéhyde par microcapsule. Les gels peuvent contenir une ou plusieurs microcapsules pour la réalisation de la préparation. Les Thalasphères (ref. SW7) ont des tailles respectives de 10 µm et 250 µm.

L'invention concerne également un procédé d'obtention des formulations à base de rétinaldéhyde qui consiste d'une part à préparer un gel aqueux à base de SEPIGEL 305 auquel on incorpore un agent antioxydant en tiédissant si nécessaire pour en faciliter la dissolution puis à ajouter le mélange d'agent conservateur et de parfum puis après complète homogénéisation les Thalasphères SW7 contenant du rétinaldéhyde dans le gel refroidi sous agitation modérée.

On peut également préparer une solution d'un agent adoucissant commercialisé sous la marque LANOL 1688 contenant l'agent antioxydant à la concentration de 0,1 %. On ajoute le mélange de parfum et d'agent conservateur puis l'agent adoucissant à la phase gélifiée. On additionne en dernier lieu des Thalasphères SW7 -sous agitation lente.

La masse fluide ainsi obtenue est répartie en tubes souples ou en pots pour assurer une parfaite conservation de la formulation.

Le parfum utilisé est un de ceux qui conviennent pour l'utilisation en dermatologie et en cosmétologie comme par exemple les essences naturelles ou synthétiques de fleurs, l'essence de rose, l'essence de violette, l'essence d'Ylang-Ylang ou l'essence de vétiver.

Les exemples suivants illustrent l'invention sans toutefois la limiter.

### Exemple I : Gel au rétinaldéhyde

| FORMULE CENTESIMALE | | |
|---|---|---|
| 1. | SEPIGEL 305 | 4 % |
| 2. | PARFUM 12220 | 0.3 % |
| 3. | GERMABEN II | 1 % |
| 4. | RETINALDEHYDE | 0.05 % |
| 5. | B.H.T. | 0.1 % |
| 6. | EAU DEMINERALISEE | 94.55 % |

### MODE DE FABRICATION

∗ Travailler à l'abri de la lumière.
∗ Peser les ingrédients séparément.
∗ Dissoudre dans l'ordre le produit 5, puis le produit 4 dans le produit 1 en chauffant très légèrement à température de 40° C.
∗ Laisser refroidir à l'abri de la lumière.
∗ Une fois que la dissolution est faite, rajouter l'eau froide dans le produit 6 en agitant continuellement, puis le produit 3 et le produit 2.

Le gel est fortement coloré en rouge.

### Exemple II : Gel au rétinaldéhyde microencapsulé

| FORMULE CENTESIMALE | | |
|---|---|---|
| A. | B.H.T. | 0.1 % |
| | SEPIGEL 305 | 4 % |
| | EAU DEMINERALISEE | 89.6 % |
| | | |
| B. | PARFUM 12220 | 0.3 % |
| | GERMABEN II | 1 % |
| | | |
| C. | THALASPHERES SW7 | |
| | chargées de rétinaldéhyde | 5 % |

### MODE OPERATOIRE

∗ Travailler à l'abri de la lumière.
∗ Préparer A, porter ce mélange à 40° C pour faciliter la dissolution du B.H.T.
∗ Ajouter rapidement l'eau à A; agiter lentement.
∗ Ajouter B, puis C en mélangeant lentement.

### Exemple III : Gel au rétinaldéhyde microencapsulé

| FORMULE CENTESIMALE | | |
|---|---|---|
| A. | EAU DEMINERALISEE | 88.6 % |
| | SEPIGEL 305 | 3 % |
| | | |
| B. | PARFUM 12220 | 0.3 % |
| | GERMABEN Il | 1 % |
| | | |
| C. | LANOL 1688 | 2 % |
| | B.H.T. | 0.1 % |
| | | |
| D. | TALASPHERES SW7 | |
| | chargées de rétinaldéhyde | 5 % |

### MODE OPERATOIRE AU LABORATOIRE

∗ Travailler à l'abri de la lumière et à froid.
∗ Préparer A en ajoutant rapidement l'eau au SEPIGEL 305.
∗ Préparer C en dissolvant le B.H.T. dans le LANOL 1688.
∗ Additionner dans l'ordre les phases B puis C dans le produit A, puis en dernier lieu les Thalasphères SW7. Agiter lentement puis laisser refroidir.
∗ On répartit le gel en flacons fermés.

Le GERMABEN II est un agent de conservation commercialisé par la firme ISP Europe Guildford (Surrey) GB. C'est un liquide visqueux transparent à peine coloré, ayant la composition :
- Diazolidinylurée : 30 %
- Méthyl paraben : 11 %
- Propyl paraben : 3 %
- Propylèneglycol : 56 %

Le LANOL 1688 est une huile émolliante commercialisée par la firme SEPPIC présentant un indice de saponification de 135 à 160, un indice de réfraction de 1,444 à 1,4465, une densité de 0,852 à 0,857, un indice d'iode < 1 et un point de congélation = - 4° C.

Le SEPIGEL 305 est un agent gélifiant commercialisé par la firme SEPPIC. C'est un mélange formé de polyacrylamide, d'isoparaffines en C₁₃ - C₁₄ et de Laureth 7.

Les Thalasphères (ref. SW7) sont des sphères commercialisées par la firme COLETICA. Les Thalasphères de 10 µm sont encapsulées dans une membrane de collagène / GAG marins. Les Thalasphères de 250 µm sont des sphères de 10 µm encapsulées dans un mélange de collagène / GAG marins contenant un pigment orange :

### Compositions des Thalasphères

0,64 % Atélocollagène marin
0,24 % Chondroïtine sulfate de sodium d'origine marine
30 % Myritol
10 % produit de charge
1 % pigment orange

Le parfum 12220 est un parfum de fleurs ayant une touche légère de roses.

## Revendications

1. Formulation galénique destinée à l'usage topique, à base de rétinaldéhyde caractérisée en ce qu'elle comporte une phase continue à consistance de gel et une phase discontinue formée de microcapsules aisément frangibles renfermant une quantité déterminée de rétinaldéhyde, dispersée d'une manière homogène dans le gel de la phase continue.

2. Formulation galénique selon la revendication 1, caractérisée en ce que la phase continue est formée d'un gel aqueux à base de (polyacrylamide, isoparaffines en C₁₃ - C₁₄ et de Laureth-7) (SEPIGEL 305).

3. Formulation galénique selon la revendication 1 ou la revendication 2, caractérisée en ce que la formulation contient en outre un agent conservateur.

4. Formulation galénique selon l'une des revendication 1 à 3, caractérisée en ce que la formulation contient en outre un agent antioxydant.

5. Formulation galénique selon l'une des revendications 1 à 4, caractérisée en ce qu'elle contient en outre un parfum.

6. Formulation galénique selon l'une des revendications 1 à 5, caractérisée en ce qu'elle contient en outre un agent adoucissant.

7. Formulation galénique selon l'une des revendications 1 à 6, caractérisée en ce que les microcapsules aisément frangibles sont des microcapsules ayant de 400 à 800 µm de long.

8. Procédé d'obtention des formulations galéniques pour usage topique selon l'une des revendications 1 à 7, qui consiste d'une part préparer un gel aqueux à base d'un agent gélifiant commercialisé sous la marque SEPIGEL 305 formé de polyacrylamide auquel on incorpore un agent antioxydant en tiédissant si nécessaire puis à ajouter le mélange formé d'un agent conservateur et d'un parfum puis après complète homogénéisation additionner des microcapsules contenant déjà le rétinaldéhyde.

9. Procédé d'obtention des formulations galéniques pour usage topique selon la revendication 8, qui consiste à disperser le parfum et l'agent conservateur dans l'agent adoucissant puis à ajouter cette dispersion à la phase gélifiée et à y additionner en dernier lieu les microcapsules contenant du rétinaldéhyde.
